(19) Europäisches Patentamt
European Patent Office
Office européen des brevets

(11) Numéro de publication: **0 258 117**
**A1**

(12) # DEMANDE DE BREVET EUROPEEN

(21) Numéro de dépôt: **87401814.6**

(22) Date de dépôt: **05.08.87**

(51) Int. Cl.⁴: **C 07 D 239/48**, C 07 C 121/75

(30) Priorité: **08.08.86 FR 8611533**

(43) Date de publication de la demande: **02.03.88 Bulletin 88/9**

(84) Etats contractants désignés: **AT BE CH DE ES FR GB GR IT LI LU NL SE**

(71) Demandeur: **SANOFI PHARMA SA, Succursale de Carouge 35, Route des Jeunes P.O. Box 150, CH-1211 Genève 26 (CH)**

(72) Inventeur: **Becker, M. Abram, 70, Hankin Street, IL-43000 Raanana (IL)**

(74) Mandataire: **Gillard, Marie-Louise et al, Cabinet Beau de Loménie 55, Rue d'Amsterdam, F-75008 Paris (FR)**

(54) **Procédé de préparation de diamino-2,4 benzyl-5 pyrimidines.**

(57) Procédé de préparation de composés de formule I, par action sur un composé de formule II, d'un alcoolate alcalin en présence d'un ester aliphatique, tel que le formiate de méthyle, puis condensation de la guanidine sur le composé ainsi obtenu de formule III.

Le composé de formule II peut être préparé par réaction de benzylaldéhyde correspondant avec l'acrylonitrile en présence de diazabicyclo-2,2,2-octane.

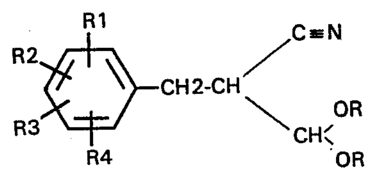

III

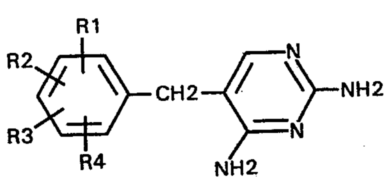

I

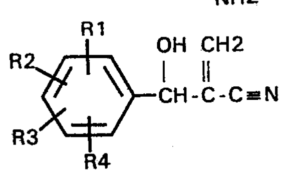

II

EP 0 258 117 A1

La présente invention concerne un nouveau procédé de préparation de diamino-2,4 benzyl-5 pyrimidines de formule I

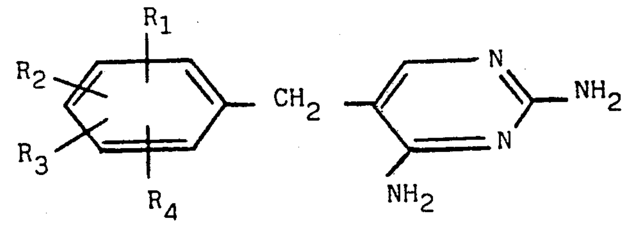

I

dans laquelle R1, R2, R3, R4, identiques ou différents, représentent l'hydrogène, un groupe alkyle en C1 à C4, un groupe alcoxy en C1 à C4, un atome d'halogène ou un groupe dialkylamino dans lequel le groupe alkyle est en C1-C3, ou R1 et R2 pris ensemble représentent un groupe méthylènedioxy.

Ces composés sont des antibactériens et, notamment, celui dans lequel R1, R2 et R3, situés en position 3,4 et 5 du noyau phényle représentent OCH3, qui est désigné par la dénomination commune internationale : triméthoprime.

De nombreux procédés de préparation des diamino-2,4 benzyl-5 pyrimidines ont été décrits. Ils correspondent, par exemple, aux schémas réactionnels suivants dans lesquels Z représente les différents substituants du noyau phényle :

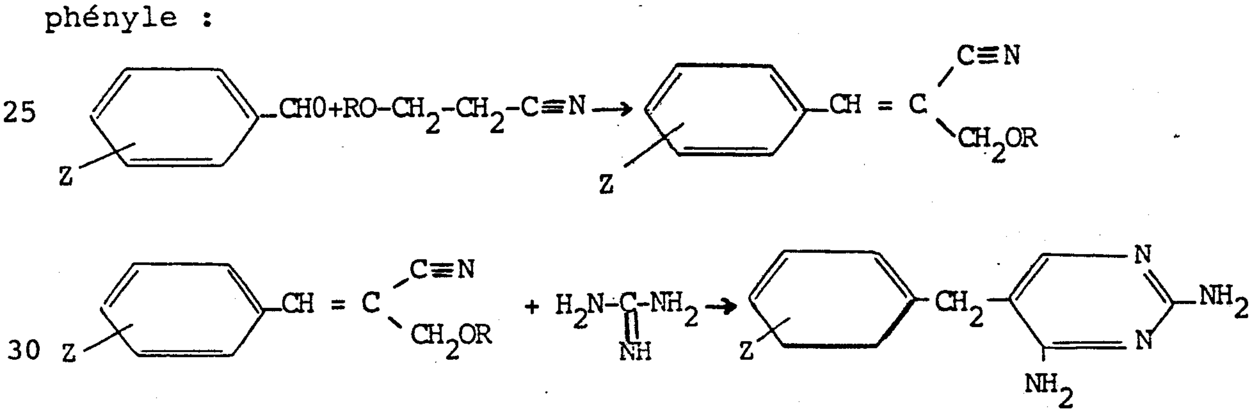

SCHEMA 1

décrit dans FR 1266428 pour R = alkyle

FR-A-2361372 pour R= O-(CH2)n-O-(CH2)m-CH3

ou

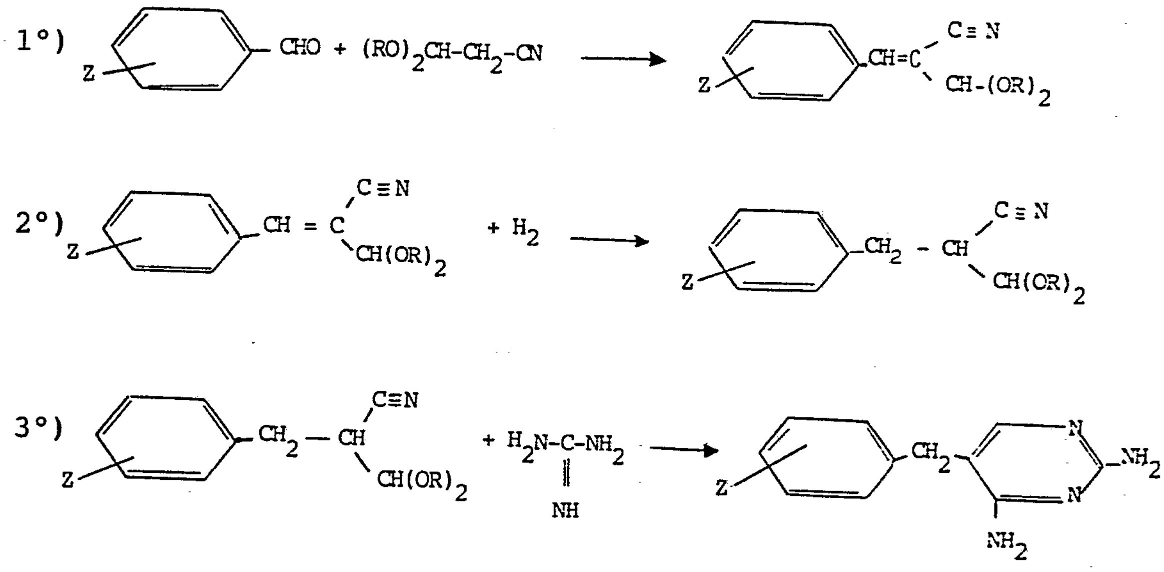


SCHEMA 2

décrit dans US 3 671 564 et FR 1 497 933.

ou

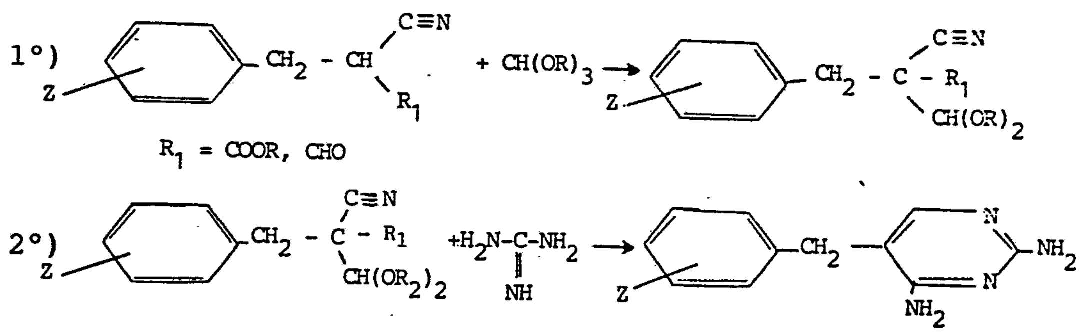


SCHEMA 3

décrit dans FR-A-2354 317

Il n'a jamais été décrit de procédé dans lequel on utilisait comme matière première un hydroxy-3 méthylène-2 phénylpropionitrile de formule II :

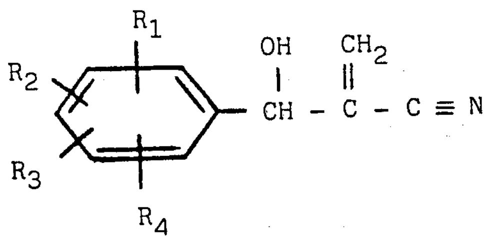

II

dans laquelle R1, R2, R3,R4 ont la même signification que précédemment.

Selon l'invention, on fait réagir les composés de formule II avec un alcoolate alcalin de formule ROM dans laquelle M est un cation alcalin, en solution dans un solvant, en présence d'un ester aliphatique pour obtenir le composé de formule III :

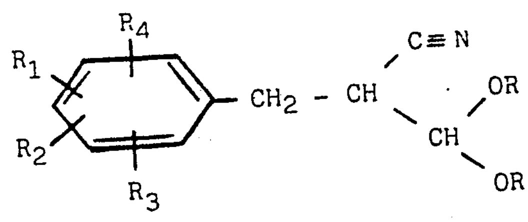

III

sur lequel on fait réagir la guanidine $H2N-\underset{NH}{\underset{\|}{C}}-NH2$ pour obtenir le composé de formule I.

L'alcoolate alcalin peut avoir de 1 à 4 atomes de carbone ; on préfère le méthylate de sodium, en particulier pour des raisons de prix de revient et de facilité de manipulation; mais on peut utiliser aussi, par exemple, l'éthylate de sodium ou un mélange de méthylate de sodium et de potassium.

On a constaté aussi que les sels alcalins, tels que ceux de potassium, accélèrent particulièrement le réarrangement en acétal III et la condensation finale avec la guanidine ; on peut en introduire dans le milieu sous forme de sel

d'un acide minéral ou organique, tel qu'un halogènure, un formiate, un acétate ou un carbonate.

Comme solvant, on préfère utiliser un solvant aprotique polaire, par exemple la N-méthylpyrrolidone et analogues, l'hexaméthylphosphotriamide ou le diméthylsulfoxyde. On préfère le diméthylsulfoxyde qui, en particulier, accélère nettement la réaction et améliore le rendement ; il peut être utilisé en mélange avec le toluène, le méthanol, ou d'autres solvants inertes. L'alcoolate peut être introduit dans le solvant, solide, ou dissous dans l'alcool correspondant.

L'ester aliphatique est un dérivé d'un alcool en C1 à C4 et d'un acide carboxylique de préférence ne comportant pas d'hydrogène sur le carbone en alpha du groupe carboxylique, tel que l'acide formique, l'acide pivalique ou l'acide oxalique ; l'acide formique est particulièrement préféré ainsi que le formiate de méthyle. On a, en effet, constaté que les esters, comme l'acétate de méthyle, n'amélioraient que peu le rendement des réactions conduisant à la formation du composé III.

On peut utiliser aussi des esters d'alkyle d'acides minéraux, inertes vis-à-vis de l'alcoolate utilisé dans la réaction comme par exemple le phosphate de triméthyle ou le silicate de tétraméthyle.

La guanidine est généralement commercialisée sous forme de carbonate ou de chlorhydrate de guanidine ; étant donné son instabilité, elle est libérée de son sel de préférence in situ ; pour cela, on peut, lorsque la réaction donnant le composé III est terminée, introduire dans le milieu réactionnel, avant le sel de guanidine, un équivalent d'alcoolate alcalin, comme le méthylate de sodium ou introduire la quantité nécessaire d'alcoolate avec celui nécessaire à la préparation de l'acétal III.

On fera en général réagir 1 à 2,5 moles d'alcoolate alcalin par mole d'hydroxy-3 méthylène-2 phénylpropionitrile en présence de 1 à 2 moles d'ester et de préférence 2 à 2,5 moles d'alcoolate. La quantité de solvant utilisée doit être suffisante pour permettre l'agitation du milieu mais pas excessive car la vitesse des réactions diminue sensiblement avec la concentration des réactifs.

Si l'on introduit un sel alcalin dans le milieu dès le début du procédé, on préfère le sel de potassium de l'acide correspondant à l'ester utilisé, à raison de 0,1 à 0,5 mole environ par mole de nitrile.

On effectue l'addition de l'alcoolate alcalin dans la solution du composé de formule II à une température comprise entre 0°C et la température ambiante, c'est-à-dire 20° - 25°C, puis le milieu réactionnel est ensuite chauffé entre 60 et 80°C ; la réaction est plus lente si on ne dépasse pas 70°C mais le produit final obtenu est alors plus facile à purifier ; la condensation du produit III avec la guanidine s'effectue à une température comprise entre 80°C et 120°C.

On pense qu'il se forme, lors de la transformation du composé de formule II en composé de formule III, des intermédiaires déjà connus, comme l'est le composé III qui est décrit notamment dans FR 1453056. En effet, lorsque la réaction du méthylate de sodium sur le composé de formule II a lieu à basse température, on peut mettre en évidence le composé de formule IV :

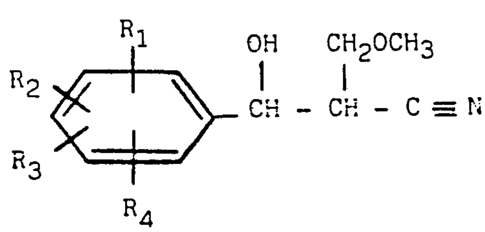

IV

qui se déshydrate spontanément en l'absence de base en cinnamonitrile de formule V :

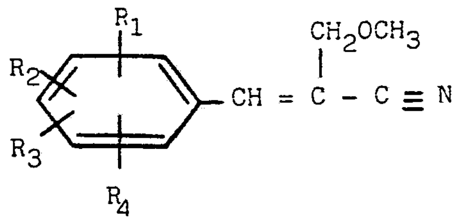

V

Le produit V est un produit connu ; on a montré qu'il ne réagissait pas avec la guanidine pour donner la diamino-2,4 benzylpyrimidine ; par contre, son isomère V' réagit facilement avec la guanidine ; dans le brevet FR 1266248, une réaction de ce type est décrite, mais le rendement obtenu dans l'exemple cité n'est que de 28 %. Aussi on préfère passer par l'intermédiaire du composé III, en utilisant un type de réaction connue ; en effet l'action du méthylate de sodium sur le composé V' conduit, après isomérisation, au composé III, connu, selon le schéma réactionnel :

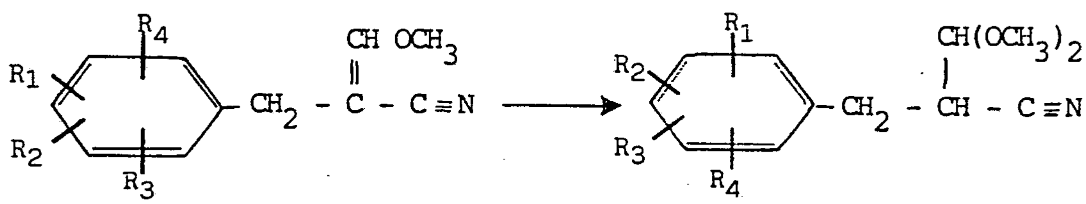

V' III

Ainsi, on a constaté que lorsque le composé III isolé et purifié est mis à réagir avec la guanidine en solution dans le butanol, à chaud, on obtient la pyrimidine brute de formule I avec 90 à 97% de rendement.

Mais on a aussi constaté que lorsqu'on fait réagir la guanidine sur le composé III sans l'isoler préalablement du milieu réactionnel où il a été préparé par le procédé de l'invention, le rendement d'obtention de la pyrimidine

par rapport au composé de formule II de départ est supérieur à 80 % et cette méthode est préférée. Si par contre la réaction de l'alcoolate alcalin sur le composé de formule II est réalisé en l'absence d'ester aliphatique, le rendement final est inférieur à 40 %, et si on fait réagir directement la guanidine et le composé de formule II dans le butanol, sans passer par l'intermédiaire du composé III, le rendement ne dépasse pas 30 %, ce qui montre le grand intérêt du procédé selon l'invention.

Le produit de départ II peut être préparé par des méthodes connues et notamment par action de l'aldéhyde :

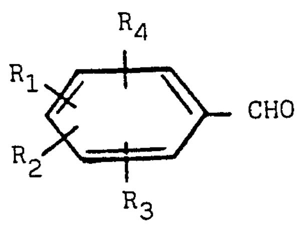

sur l'acrylonitrile $CH2 = CH - C \equiv N$ en présence d'un catalyseur convenablement choisi. On a notamment décrit que la réaction d'un aldéhyde Z-CHO avec l'acrylonitrile catalysée, dans GB-1 168 000, par des phosphines, ou dans US 3,743,669, par des amines tertiaires tricycliques, telles que la quinuclidine, la triéthylènediamine, ou le diazabicyclo-2,2,2-octane (dénommé ci-après DABCO), permettait d'obtenir des acrylonitriles alpha-hydroxylés de formule :

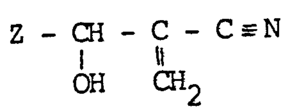

Cependant, les exemples mentionnés dans les deux références ci-dessus ne concernent que des aldéhydes aliphatiques et la demanderesse a constaté que les rendements de la synthèse étaient nettement inférieurs pour les aldéhydes

aromatiques, en particulier si l'on utilise les conditions opératoires indiquées dans le brevet US 3,743,669.

En effet, lorsque l'on met du triméthoxybenzaldéhyde en solution dans l'acrylonitrile et que l'on ajoute une quantité catalytique de DABCO (0,1 mole pour une mole d'aldéhyde), on obtient après une semaine à température ambiante le produit de formule II avec 50 à 60% de rendement seulement, tandis que si l'on chauffe le milieu réactionnel, il se forme de nombreux produits secondaires sans que le rendement soit nettement plus élevé.

Un autre objet de l'invention est donc un procédé amélioré de préparation du composé de formule II, procédé par lequel on obtient ce composé à partir de l'aldéhyde et de l'acrylonitrile avec un rendement voisin du maximum, et en tout cas supérieur à 90%. Ce procédé met en jeu un intermédiaire de synthèse qui n'a jamais été isolé, et qui est un complexe constitué de deux molécules du composé de formule II et d'une molécule de DABCO.

Ces complexes cristallins sont stables ; ils peuvent être recristallisés et les analyses qui ont été effectuées (analyse élémentaire, spectres IR et RMN) ont confirmé leur structure. Il sont décomposés par réaction avec un acide, en donnant le sel du DABCO et le composé de formule II. Ces complexes sont un autre objet de l'invention ainsi que les composés de formule II préparés par leur intermédiaire et en particulier :

- le complexe de formule :

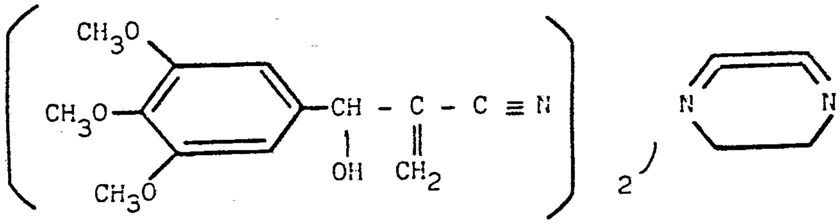

dont le point de fusion est de 111-112°C après recristallisation dans le toluène ou l'acétate d'isopropyle;

- le complexe de formule :

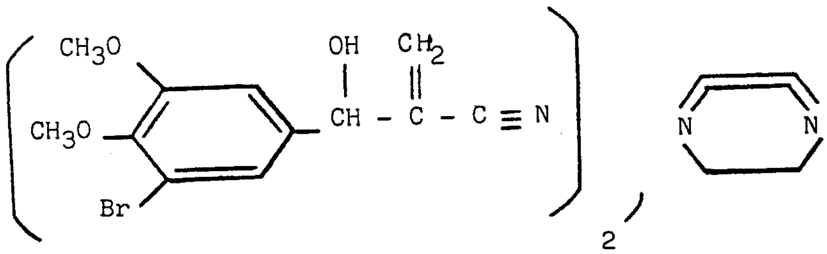

qui a un point de fusion de 94-96°C ;

- le complexe de formule :

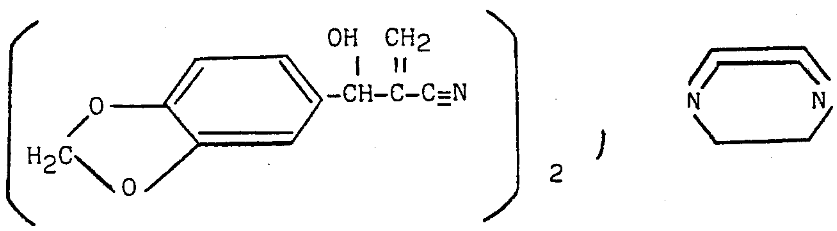

qui a un point de fusion de 89-91°C ; ou

- le complexe de formule :

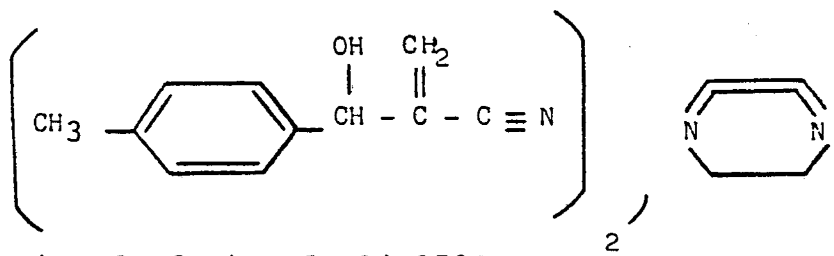

qui a un point de fusion de 94-95°C.

Ces complexes se forment lorsque l'on fait réagir de 0,5 mole à 1 mole de DABCO sur 1 mole d'aldéhyde en solution dans de l'acrylonitrile. On préfère utiliser de 0,5 à 0,6 mole de DABCO. Dans certains cas, il est nécessaire d'utiliser un net excès d'acrylonitrile pour éviter que le milieu ne prenne en masse. On peut aussi opérer avec seulement 1,2 à 1,5 équivalents d'acrylonitrile, en présence d'un tiers solvant convenablement choisi dans lequel le complexe précipitera. Avec les solvants comme le toluène, le cyclohexane, le chloroforme, le chlorure de méthylène, l'acétate d'éthyle, l'acétonitrile, le

diméthylformamide , le méthanol ou l'éthanol, les réactions sont très lentes et les rendements assez faibles, avec une formation plus ou moins importante de produits secondaires. Par contre l'alcool tertiobutylique, les solvants à fonction éther, tels que le dioxanne, le tétrahydrofuranne, le diméthoxyméthane, et les éthers de l'éthylène glycol ou du diéthylène glycol donnent des résultats satisfaisants.Ces solvants peuvent être utilisés purs, en mélange en proportions variables entre eux ou avec moins de 20% d'un solvant protique, ce qui augmente la vitesse de la réaction. Le solvant protique peut être choisi parmi les solvants hydroxylés et les amides primaires. A titre d'exemples de solvants hydroxylés, on peut citer notamment les alcools purs ou aqueux en C1-C4, tels que le méthanol, l'éthanol ou l'éthanol aqueux à 95 % et comme amides primaires le formamide ou l'acétamide.

La réaction est effectuée de préférence à température ambiante, ce qui évite la formation de produits secondaires.

En fin de réaction, on peut soit isoler par filtration le complexe formé, soit ajouter dans le milieu une solution aqueuse acide pour décomposer immédiatement ledit complexe,soit le transformer in situ en cinnamonitrile de formule V, à partir duquel on prépare ensuite le composé de formule III selon un procédé connu ; lorsque le milieu contient un solvant non miscible à l'eau, on peut séparer les deux phases ; la phase aqueuse contient le sel de DABCO avec l'acide, d'où le DABCO lui-même pourra être reprécipité pour être recyclé dans une opération ultérieure, sans purification particulière. La phase organique, ne contenant plus de DABCO sera concentrée sous vide pour éliminer le solvant et l'acrylonitrile n'ayant pas réagi.

Lorsque l'on prépare le composé de formule II par le procédé mettant en oeuvre la formation du complexe de DABCO, la diaminopyrimidine pure de formule I est préparée avec 80 à 85 % de rendement par rapport à l'aldéhyde de départ en appliquant le procédé selon l'invention ; le rendement est nettement supérieur aux rendements obtenus en appliquant les procédés précédemment décrits.

Dans ce qui suit, des exemples de réalisation de l'invention sont décrits.

EXEMPLE 1

Préparation de l'hydroxy-3 méthylène-2 (triméthoxy-3,4,5 phényl)-3 propionitrile.

a) 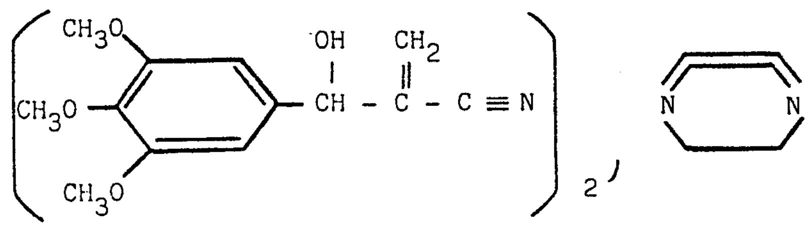

Dans un réacteur de 500 ml muni d'un système d'agitation, on introduit 55 ml d'acrylonitrile, 50 ml de diméthoxyméthane, 20 g de formamide, 100 g de (triméthoxy - 3,4,5) benzaldéhyde et 30 g de DABCO commercial ou la quantité équivalente de DABCO récupéré à la fin d'une opération précédente. Un précipité apparaît au bout de quelques heures et la réaction est terminée après 20 à 36 heures d'agitation.

On isole alors le complexe par filtration du milieu réactionnel ; un second jet est récupéré après concentration du filtrat ; le rendement est alors supérieur à 90%.

On obtient le même complexe en laissant sous agitation, vers 20°C, 196 g de (triméthoxy -3,4,5) benzaldéhyde, 300 g d'acrylonitrile et 60 g de DABCO pendant 24 heures. Le complexe précipite dans le milieu ; on isole un second jet par addition d'éther éthylique au filtrat.

Le complexe après recristallisation dans trois volumes de toluène a un point de fusion de 111 - 112°C. Les spectres RMN et IR, qui sont conformes, montrent que les hydroxyles sont engagés dans des liaisons hydrogènes.

b) libération du nitrile de son complexe

Le complexe est dissous dans quatre volumes de dichloroéthane-1,2, et la phase organique est lavée par une solution aqueuse d'acide chlorhydrique 5N jusqu'à pH 4-4,5 de la phase aqueuse.

Les deux phases sont alors séparées ; la phase organique est lavée avec 100 ml d'eau, puis le solvant est éliminé sous pression réduite. Le résidu épais, prend en masse ; c'est de l'hydroxy-3 méthylène-2 (triméthoxy-3,4,5 phényl)-3 propionitrile. Après recristallisation dans un mélange toluène/heptane (1/1), le produit fond à 77 - 79°C.

Spectre RMN (CDCl3, TMS): 6,68 (s, 2H) ; 6,12 (dd, 2H) ; 5,25 (s,1H) ; 3,85 (deux s, 9H) ; 3,18 (1H échangeable).

EXEMPLE 2

Préparation de l'hydroxy-3 méthylène-2 (triméthoxy-3,4,5 phényl)-3 propionitrile sans isoler le complexe.

On opère comme dans l'exemple 1-a mais en fin de réaction au lieu de filtrer le milieu réactionnel, on ajoute 300 ml de dichloro-1,2 éthane et 100 ml de HCl aqueux 5N. On décante alors la phase aqueuse, et la lave deux fois avec 50 ml de dichloro-1,2 éthane.

Les phases organiques, lavées à l'eau, sont réunies et le solvant évaporé sous pression réduite. On obtient ainsi à partir de 100 g d'aldéhyde, 127,5 g d'hydroxy-3 méthylène-2 (triméthoxy-3,4,5 phényl)-3 propionitrile.

La solution aqueuse chlorhydrique contenant le DABCO est concentrée par évaporation sous pression réduite ; lorsque la teneur en eau n'est plus que de 30 %, on refroidit le mélange et on ajoute une quantité suffisante de solution de NaOH dans le méthanol ( c = 10 % p/v) pour libérer le DABCO de son sel ; après filtration du chlorure de sodium, on élimine le méthanol sous pression réduite. Le DABCO ainsi récupéré quantitativement est réutilisable pour une opération ultérieure sans purification supplémentaire.

EXEMPLE 3

Complexe de l'hydroxy-3 méthylène-2 (méthylène dioxy-3,4 phényl)-3 propionitrile avec le DABCO.

On agite à la température ambiante 15 g de (méthylènedioxy-3,4)benzaldéhyde avec 5,6 g de DABCO et 20 ml d'acrylonitrile, pendant 48 heures ; on constate , par chromatographie en couches minces, qu'il n'y a plus de benzaldéhyde de départ. On refroidit à -10°C et filtre pour isoler le précipité. Celui-ci est lavé au toluène et séché. On isole ainsi 22 g de complexe. Un second jet de 3,5 g peut être obtenu après concentration des eaux mères. Le rendement est de 95 % environ. Le complexe peut être recristallisé dans le toluène ou l'acétate d'isopropyle.Pur, il fond à 89-91°C et est formé de 2 molécules de nitrile pour une de DABCO.

EXEMPLE 4

Préparation de la diamino-2,4 (triméthoxy-3,4,5 benzyl)-5 pyrimidine.

Dans un réacteur de 1 litre, on introduit 125,5 g du nitrile précédemment préparé avec 150 ml de toluène, 53 ml de formiate de méthyle et 50 ml de diméthylsulfoxyde. Le mélange est refroidi à 0°C et on ajoute peu à peu 210 ml d'une solution a 30% (p/v) de méthylate de sodium dans le méthanol, en évitant que la température s'élève au delà de 20°C ; on porte ensuite le milieu réactionnel lentement à 65 - 70°C et on le maintient environ 16 à 18 heures à cette température. On refroidit alors jusqu'à 30°C et ajoute 120 ml de méthanol puis 55 g de carbonate de guanidine. Le milieu réactionnel est alors de nouveau chauffé et on distille le méthanol et du toluène jusqu'à ce que le milieu ait une température interne de 110°C environ. Après une heure trente à cette température la cyclisation est terminée ; on refroidit jusque vers 100°C et le produit final brut précipite. Le précipité isolé est lavé à l'eau puis à l'acétone ou au dichloréthane. On obtient ainsi 121,3 g de diamino-2,4 (triméthoxy-3,4,5 benzyl)-5 pyrimidine. Après purification par les méthodes classiques, on obtient 118 g de produit pur, soit un rendement de 79,8% par rapport au triméthoxybenzaldéhyde de départ, lorsque l'on a préparé le nitrile par la méthode décrite à l'exemple 2.

EXEMPLE 5

Préparation de la diamino-2,4(triméthoxy-3,4,5 benzyl)-5 pyrimidine.

Dans un réacteur d'un litre, on introduit 125,5 g du nitrile avec 150 ml de toluène, 20 g de formiate de potassium, 53 ml de formiate de méthyle et 50 ml de diméthylsulfoxyde ; on ajoute 230 ml de solution à 30% de méthylate de sodium dans le méthanol en maintenant la température à moins de 20°C, puis on maintient le milieu réactionnel à 65°C environ pendant 10 à 12 heures. On ajoute ensuite à 30°C environ, 64 g de carbonate de guanidine. Le milieu réactionnel est ensuite traité comme précédemment. On isole finalement 125 g de produit final brut qui donnent 121,4 g de produit pur.

EXEMPLE 6

Préparation du (triméthoxy-3,4,5 phényl)-3 méthoxyméthyl-2 acrylonitrile.

On introduit dans un réacteur de 500 ml muni d'un système d'agitation 55 ml d'acrylonitrile, 50 ml de diméthoxyméthane, 100 g de (triméthoxy-3,4,5) benzaldéhyde et 30 g de DABCO. Après 25 heures d'agitation, on ajoute 50 g de formiate de méthyle et 100 ml de méthanol et on agite à température ambiante plus de 24 heures jusqu'à disparition de l'hydroxy-3 méthylène-2 (triméthoxy-3,4,5) phényl-3 propionitrile ; le (triméthoxy-3,4,5 phényl)-3 méthoxyméthyl-2 acrylonitrile formé est isolé ou mis à réagir avec le méthylate de sodium après élimination des solvants et réactifs volatils pour donner l'acétal de formule III.

## REVENDICATIONS

1. Procédé de préparation de diamino-2,4 benzyl-5 pyrimidines de formule I :

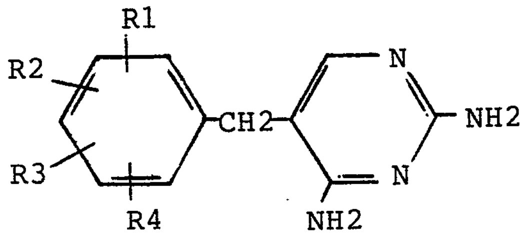

I

dans laquelle R1, R2, R3 et R4, identiques ou différents, représentent l'hydrogène, un groupe alkyle en C1-C4, un groupe alcoxy en C1-C4, un groupe dialkylamino dans lequel le groupe alkyle est en C1-C3 ou un atome d'halogène ou R1 et R2 pris ensemble, représentent un groupe méthylènedioxy, caractérisé en ce que l'on fait réagir un hydroxy-3 méthylène-2 phénylpropionitrile de formule II :

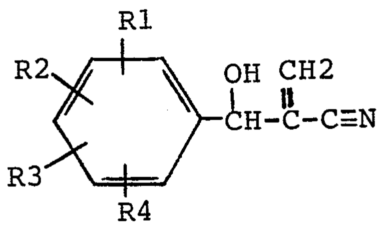

II

dans laquelle R1, R2, R3 et R4 ont la même signification que précédemment, avec un alcoolate alcalin de formule ROM dans laquelle M représente un cation alcalin et R est un groupe alkyle en C1 à C4, dans un solvant et en présence d'un ester d'alcool aliphatique pour obtenir le composé de formule III :

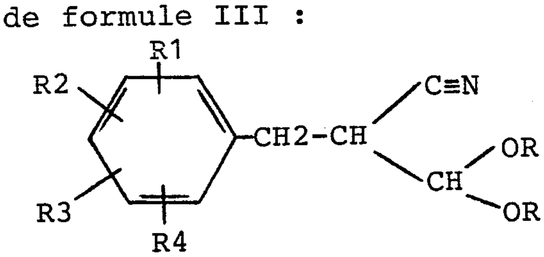

III

sur lequel on fait réagir la guanidine.

2. Procédé selon la revendication 1, caractérisé en ce que l'on utilise au moins une mole d'ester par mole de propionitrile de formule II.

3. Procédé selon l'une des revendications 1 ou 2, caractérisé en ce que le solvant est un solvant aprotique polaire.

4. Procédé selon l'une quelconque des revendications 1 à 3, caractérisé en ce que le solvant est un mélange de diméthylsulfoxyde et de méthanol.

5. Procédé selon l'une quelconque des revendications 1 à 3, caractérisé en ce que le solvant est un mélange de diméthylsulfoxyde, de toluène et de méthanol.

6. Procédé selon l'une quelconque des revendications 1 à 5, caractérisé en ce que l'ester est un dérivé d'un alcool en C1 à C4 et d'un acide carboxylique choisi parmi l'acide formique, l'acide pivalique et l'acide oxalique.

7. Procédé selon la revendication 6, caractérisé en ce que l'ester est le formiate de méthyle.

8. Procédé selon l'une quelconque des revendications 1 à 7, caractérisé en ce qu'on introduit avec l'ester, un sel alcalin d'acide carboxylique.

9. Procédé selon l'une quelconque des revendications 1 à 8, caractérisé en ce que la guanidine est libérée de l'un de ses sels in situ, par action d'un alcoolate alcalin.

10. Procédé selon l'une quelconque des revendications 1 à 9, caractérisé en ce que l'alcoolate est le méthylate de sodium.

11. Procédé selon l'une quelconque des revendications 1 à 10, caractérisé en ce que le composé de formule I est la diamino-2,4 (triméthoxy-3,4,5 benzyl)-5 pyrimidine.

12. Procédé selon l'une quelconque des revendications 1 à 11, caractérisé en ce que le composé de formule II est obtenu par réaction de l'aldéhyde de formule :

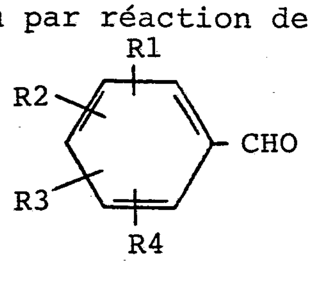

dans laquelle R1, R2, R3 et R4 sont tels que définis dans la revendication 1, et de l'acrylonitrile $CH2=CH-C\equiv N$ en présence de 0,5 à 1 équivalent, par rapport à l'aldéhyde, de diazabicyclo-2,2,2-octane.

13. Procédé selon la revendication 12, caractérisé en ce qu'on utilise de 0,5 à 0,6 équivalent de diazabicyclo-2,2,2-octane.

14. Procédé selon l'une des revendications 12 ou 13, caractérisé en ce que la réaction d'addition est effectuée dans un solvant choisi parmi le dioxanne, le tétrahydrofuranne, le diméthoxyméthane, les éthers de l'éthylène glycol et du diéthylène-glycol et le tertio-butanol.

15. Procédé selon l'une quelconque des revendications 12 à 14, caractérisé en ce que le solvant est le diméthoxyméthane.

16. Procédé selon l'une quelconque des revendications 12 à 15, caractérisé en ce que le milieu réactionnel contient en outre un solvant protique choisi parmi les solvants hydroxylés, et les amides primaires.

17. Procédé selon la revendication 16, caractérisé en ce que l'on utilise un alcool en C1 à C4.

18. Procédé selon l'une des revendications 1 à 11, caractérisé en ce que le composé de formule III est obtenu sans isoler le nitrile de formule II préparé par le procédé décrit dans l'une des revendications 12 à 17.

19. Complexes de formule :

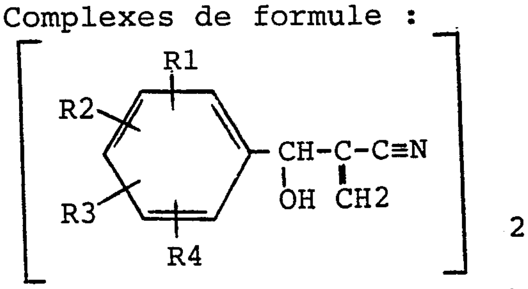
,
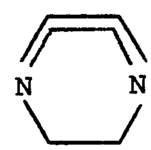

dans laquelle R1, R2, R3 et R4, identiques ou différents représentent l'hydrogène, un groupe alkyle en C1 à C4, un groupe alcoxy en C1 à C4, un atome d'halogène, un groupe dialkylamino dans lequel le groupe alkyle est en C1 à C3, ou R1 et R2 pris ensemble, représentent un groupe méthylènedioxy.

20. Complexe selon la revendication 19, caractérisé en ce qu'il répond à la formule :

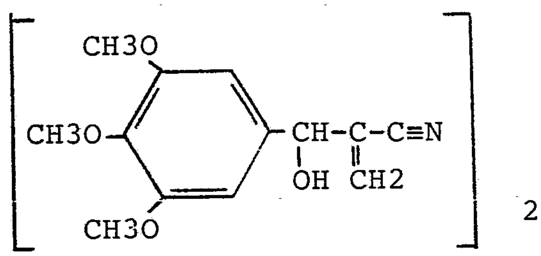 , 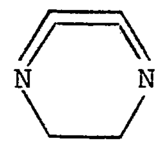

21. Procédé pour l'obtention d'hydroxypropionitriles de formule II :

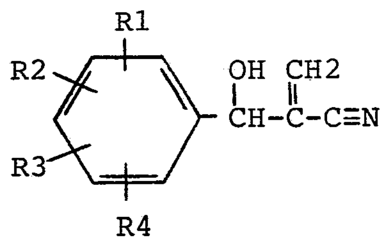

II

dans laquelle R1, R2, R3 et R4 sont tels que définis dans la revendication 1, caractérisé en ce qu'il consiste à faire réagir une mole d'aldéhyde de formule :

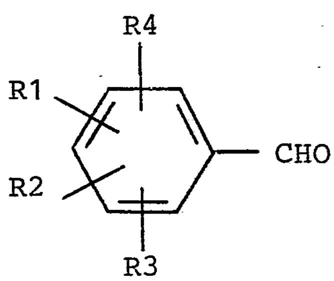

en solution dans l'acétonitrile CH2 = CH - C = N éventuellement en présence d'un tiers solvant avec 0,5 à 1 mole de DABCO, pour former un complexe selon l'une des revendications 18 ou 19, que l'on décompose par action d'un acide pour former l'hydroxypropionitrile de formule II.

22. Hydroxy-3 méthylène-2 (triméthoxy-3,4,5 phényl)-3 propionitrile.

0258117

Office européen des brevets

# RAPPORT DE RECHERCHE EUROPEENNE

Numero de la demande

EP 87 40 1814

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | ANGEW. CHEM. INT. ED. ENGL., vol. 22, no. 10, 1983, page 795, Verlag Chemie GmbH, Weinheim, DE; H.M.R. HOFFMANN et al.: "Preparation of 2-(1-hydroxyalkyl)acrylic esters; simple three-step synthesis of mikanecic acid" * Page 795 * --- | 1,12,13,21,22 | C 07 D 239/48<br>C 07 C 121/75 |
| A | FR-A-2 508 450 (EGYT) * Pages 1-9, en particulier exemple 1 * --- | 1,10,11 | |
| A | GB-A-1 153 630 (TOYO RAYON) * Pages 1-8 * ----- | 1,3,21,22 | |
| | | | **DOMAINES TECHNIQUES RECHERCHES (Int. Cl.4)** |
| | | | C 07 D 239/00<br>C 07 C 121/00 |

Le présent rapport a été établi pour toutes les revendications

| Lieu de la recherche | Date d'achèvement de la recherche | Examinateur |
|---|---|---|
| LA HAYE | 05-11-1987 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)